# EUROPEAN PATENT SPECIFICATION

(11) **EP 3 536 222 B1**
(45) Date of publication and mention of the grant of the patent: **21.08.2024**
(21) Application number: 19158476.2
(22) Date of filing: 21.02.2019
(51) Int. Cl.: A61B 3/10, G01B 9/02

(54) **OCT APPARATUS**
OCT-VORRICHTUNG
APPAREIL DE TOMOGRAPHIE PAR COHÉRENCE OPTIQUE (OCT)

(30) Priority: 06.03.2018 JP 2018039178
(43) Date of publication of application: 11.09.2019
(73) Proprietor: Nidek Co., Ltd., Gamagori-shi, Aichi, 443-0038 (JP)
(72) Inventor: ITO, Koichi, Aichi (JP); FUJIU, Kenshiro, Aichi (JP)
(74) Representative: Hoefer & Partner Patentanwälte mbB

(56) References cited:
- EP-A1- 2 141 447
- EP-A1- 2 497 413
- EP-A2- 3 446 620
- WO-A1-2016/111379
- WO-A2-2015/091796
- JP-A- 2017 086 311
- US-A1- 2012 281 235
- US-A1- 2014 268 048
- US-A1- 2017 332 898

## Description

### TECHNICAL FIELD

The present disclosure relates to an OCT apparatus for obtaining OCT data of a fundus of a subject eye.

### BACKGROUND

As an OCT apparatus for obtaining OCT data of a fundus of a subject eye, for example, an apparatus capable of acquiring the OCT data by processing a spectrum interference signal output from an OCT optical system is known. In recent years, an apparatus for obtaining fundus OCT data at a wide angle has been proposed.

For example, JP-A-2016-123467 discloses a fundus imaging apparatus. In the fundus imaging apparatus, a main body has an optical system for scanning the fundus, and an attachment optical system is inserted between the main body and the subject eye, and then, a scanning range of measurement light on the fundus is expanded compared to a case when the attachment optical system is retracted (not inserted).

When obtaining fundus OCT data at a wide angle, for example, there is a possibility that a peripheral area of the fundus cannot be observed due to insufficient imaging range in the depth direction.
From WO 2016/111379 A1, an optical tomographic imaging apparatus according to the preamble of claim 1 is known which includes
a determination unit for determining whether or not an optical member for changing a field angle has been inserted between a scanning unit and an object to be inspected to change a field angle of an acquiring area of a tomographic image.
From EP 3 446 620 A2, an OCT optical system is known which includes a compensation unit that compensates for an amount of change in a light path of a measurement light path between a state in which an angle-of-view switching optical system is inserted and a state in which the angle-of-view switching optical system is retracted.
From JP 2017 086311 A, an OCT apparatus is known which includes a switching means for toggling between a mode of a reference light bypassing a light path length changing material and a mode of the reference light passing through the light path length changing material to switch the light path length of the reference light; and image generation means for generating a new tomographic image based on a tomographic image captured in the mode of the reference light bypassing the light path length changing material in a first range and a tomographic image captured in the mode of the reference light passing through the light path length changing material in a second range different from the first range.
From US 2017/332898 A1, an OCT apparatus is known which includes a focus adjustment with respect to an object to be inspected even when an optical member for changing a field angle is inserted in order to change the field angle of an acquiring area of a tomographic image for acquiring a clear tomographic image with a focus on the object to be inspected.
From US 2012/281235 A1, an OCT apparatus is known which includes a display controller for displaying a tomographic image and identifying information used to determine whether the tomographic image output to the monitor is a normal image or a reverse image.

### SUMMARY

An object of this disclosure is to provide an OCT apparatus that is capable of easily observing OCT data at a wide angle.

The solution of this object is solved by the features of the independent claim. The dependent claims contain advantageous embodiments of the present invention.

The fundus OCT data at a wide angle can be easily observed.

### BRIEF DESCRIPTION OF DRAWINGS

Fig. 1 is a diagram illustrating an example of an optical system of an OCT apparatus according to the present embodiment.
Fig. 2 is a diagram illustrating an example of a control system of the OCT apparatus according to the present embodiment.
Fig. 3A is a diagram illustrating an example of an image shown by OCT data in the present embodiment (normal imaging), and Fig. 3B is a diagram illustrating an example of output on the display unit in the present embodiment (normal imaging).
Fig. 4A is a diagram illustrating an example of an image shown by OCT data in the present embodiment (wide-angle imaging), and Fig. 4B is a diagram illustrating an example of output on the display unit in the present embodiment (wide-angle imaging).

### DETAILED DESCRIPTION

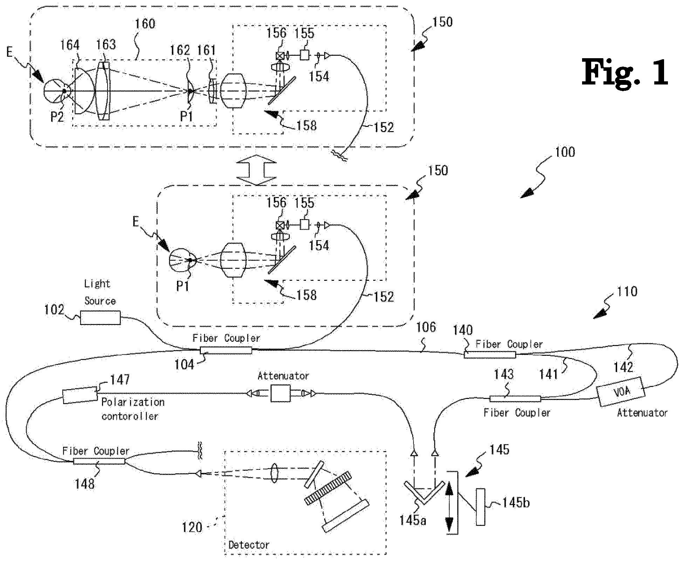

An example of an embodiment of the present disclosure will be described with reference to the drawings. Fig. 1 to Fig. 4B are diagrams relating to the embodiment of the present embodiment. Items classified by the following < > can be used independently or in association with each other.

An OCT apparatus according to the embodiment may include an OCT optical system and may be capable of processing a spectral interference signal output from a detector of the OCT optical system and acquiring OCT data. In this case, the OCT optical system may be a Fourier domain OCT optical system (an SS-OCT optical system or an SD-OCT optical system), for example. The OCT optical system may have a light splitter for splitting light from an OCT light source to light traveling to a measurement light path and light traveling to a reference light path and may detect the spectral interference signal of measurement light guided via the measurement light path to a subject and reference light from the reference light path.

In addition, the OCT apparatus may include an image processor, and the image processor may be capable of processing a spectral interference signal output from the OCT optical system and acquiring the OCT data.

### <Wide-angle Imaging of Fundus>

For example, the OCT optical system may have a light splitter, which splits light from an OCT light source to light traveling to the measurement light path and light traveling to the reference light path, and a detector, which detects an interference signal of the measurement light guided to a fundus of a subject eye via the measurement light path and the reference light from the reference light path.

The OCT optical system may be an OCT optical system that is capable of guiding the measurement light to a wide-angle region. The wide-angle region includes a center area (fundus center area) and a peripheral area (fundus peripheral area) of the fundus in one transverse direction in which the measurement light traverses the fundus. For example, in a case where the measurement light traverses the fundus in a specific transverse direction (for example, a horizontal direction), the wide-angle region may a region at a wide angle so as to traverse both of the fundus center area and the fundus peripheral area. In addition, regarding a transverse region that the measurement light traverses, a transverse region in the fundus center area and a transverse region in the fundus peripheral area may be continuous to each other in the transverse direction, for example. For example, a region having a length of 18 mm or longer on the fundus may be set as the wide-angle region. It is needless to say that the wide-angle region may be used in a case of obtaining a region narrower than 18 mm. The apparatus of the embodiment is used particularly in a case of imaging a peripheral region of a subject eye of which a fundus has a large degree of curvature.

For example, at least a region including a macular region and an optic nerve head of the fundus may be set as the fundus center area, and a region including both regions on outer sides from both end portions of the fundus center area in one transverse direction may be set as the fundus peripheral area. It is needless to say that setting is not limited to this, and at least a region including the macular region of the fundus may be set as the fundus center area, and a region including both regions on outer sides from both end portions of the fundus center area in one transverse direction may be set as the fundus peripheral area, for example.

For example, the OCT optical system that is capable of guiding the measurement light to the wide-angle region of the fundus may include an objective lens optical system or may include an objective mirror optical system using a concave mirror. In addition, in the OCT optical system, an attachment optical system may be attached to (inserted into) the objective lens optical system.

### <Switching of Display States>

An OCT apparatus may include a controller as a control unit. The controller may be, for example, a controller that can output OCT data obtained based on a spectrum interference signal to a display unit. In addition, the controller may control an OCT optical system and obtain the OCT data, for example.

As imaging modes for obtaining the OCT data, a first imaging mode for obtaining the OCT data on a fundus center area of the subject eye and a second imaging mode for obtaining the OCT data on a wide-angle region including the fundus center area and a fundus peripheral area of the subject eye, may be set.

The controller may switch the display state of the display unit between the first imaging mode for obtaining the OCT data on the fundus center area and the second imaging mode for obtaining the OCT data on the wide-angle region including the fundus center area and the fundus peripheral area. In this way, it is possible to easily observe the OCT data on the wide-angle region.

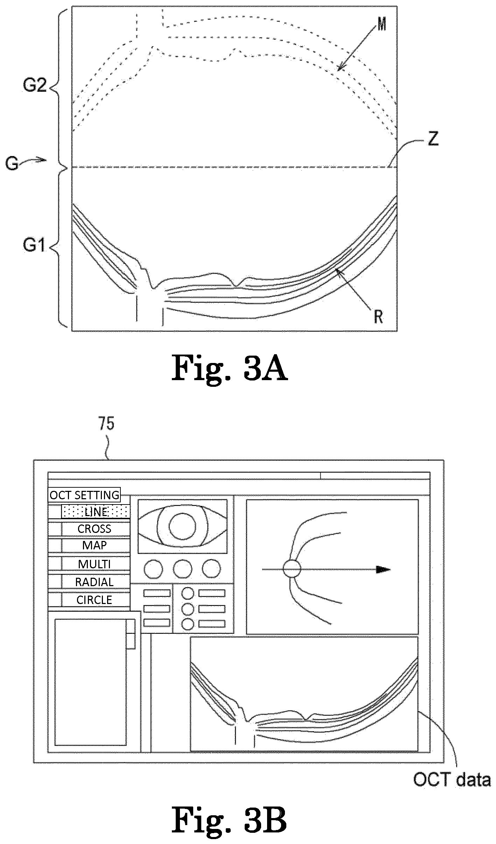

When switching the display state, the controller may change an output range of the OCT data on the screen of the display unit (refer to, for example, Figs. 3A, 3B, s4A and 4B). For example, in a case where the first imaging mode is set, the controller outputs OCT data of any one of the front and rear image areas with respect to the zero delay position on an image shown by the OCT data may be output to the display unit, and in a case where the second imaging mode is set, the controller outputs OCT data of both the front and rear image areas with respect to the zero delay position on an image shown by the OCT data may be output to the display unit. In this way, the OCT data on the fundus center area and the OCT data on the wide-angle region can be appropriately observed, respectively. Of course, not limited to the above, for example, when the mode is set as the first imaging mode, the entire image areas of the front and rear of the OCT data and a part of the other image area with respect to the zero delay position on an image shown by the OCT data may be output to the display unit, and when the mode is set as the second imaging mode, both the front and rear image areas with respect to the zero delay position on an image shown by the OCT data may be output to the display unit.

In the case where the controller outputs OCT data of both the front and rear image areas with respect to the zero delay position on an image shown by the OCT data are output to the display unit, the controller may process to display an image shown by the OCT data including both a real image and a virtual image on the display unit. In this case, after processing for removing any one of the real image and the virtual image is performed, one of the images may be displayed on the display unit.

The controller may synthesize the OCT data on the fundus center area acquired in the first imaging mode and the OCT data on the wide-angle region including the fundus center area and the fundus peripheral area acquired in the second imaging mode, and then, may display the composite OCT data on the display unit. According to this, since the image quality of OCT data of the fundus center area in the OCT data on the wide-angle region can be improved, it is possible to achieve a more accurate observation.

The switching of the mode between the first imaging mode and the second imaging mode may be performed by a manual operation of an examiner or may be automatically performed. Furthermore, the controller may switch the display state of the display unit according to the switching of the imaging mode.

For example, the controller may switch the display state of the display unit according to switching of the imaging mode by insertion and retraction of a wide-angle attachment to the OCT optical system. In addition, according to the switching of the imaging mode by changing the scanning range of the measurement light onto the fundus, the display state of the display unit may be switched.

For example, if the scanning range of the measurement light onto the fundus is within a predetermined range, the controller may be set to the first imaging mode, and if the scanning range of the measurement light onto the fundus exceeds the predetermined range, may be set to the second imaging mode. The controller may switch the output range of the OCT data to the screen of the display unit with the mode switching signal of imaging mode as a trigger.

In this way, it is possible to smoothly perform the switching of the display state of the display unit according to the imaging mode. It is possible to smoothly perform the observation of each OCT data.

In the description above, the display state of the display unit is switched in accordance with the imaging mode, but not limited thereto. For example, the controller may be able to switch a mode between a first display mode in which the controller outputs OCT data of any one of the front and rear image areas with respect to the zero delay position on an image shown by the OCT data is output to the display unit and a second display mode in which the controller outputs OCT data of both the front and rear image areas with respect to the zero delay position on an image shown by the OCT data is output to the display unit. In switching the mode between the first display mode and the second display mode, a method similar to the method of switching the mode between the first imaging mode and the second imaging mode may be used. In addition, a switching method described in the example described later may be used.

### <Dispersion Correction by Software>

The OCT apparatus may include a storage unit. For example, the storage unit may be a storage unit for storing dispersion correction data for correcting the dispersion in the OCT data. The controller may correct the spectrum interference signal using the dispersion correction data obtained from the storage unit, and may perform a Fourier analysis of the corrected spectrum interference signal to acquire the OCT data. In this way, it is possible to distinguish the real image and the virtual image, even when both the front and rear image areas with respect to the zero delay position are output to the display unit, it is possible to easily perform the observation of the OCT data.

### <Analysis Processing>

The OCT apparatus may include an analysis processing unit. The analysis processing unit may be, for example, an analysis processing unit which analyzes the OCT data to obtain an analysis result. The controller described above may serve as the analysis processing unit, or the analysis processing unit may be separately provided from the controller.

When analyzing the OCT data obtained in the first imaging mode, the analysis processing unit may acquire the analysis result by analyzing OCT data of any one of the front and rear image areas with respect to the zero delay position on an image shown by the OCT data. When analyzing the OCT data acquired in the second imaging mode, the analysis processing unit may acquire the analysis result by analyzing OCT data of both the front and rear image areas with respect to the zero delay position on an image shown by the OCT data. In this way, it is possible to perform the analysis processing according to the imaging mode of the acquired OCT data.

An example of an embodiment of the OCT optical system for obtaining the OCT data of the wide-angle region will be described below. Of course, the OCT optical system according to the present embodiment is not limited to the following configuration.

### <Angle-of-view Switching Optical System>

Hereinafter, a case where an angle-of-view switching optical system can be inserted into and removed from a light guiding optical system disposed on the measurement light path, and a size of an angle of view, which indicates a scanning range of the measurement light on the fundus is different between an insertion state and a retraction state will be described. In this case, in the embodiment, the size of the angle of view is more increased in the insertion state than in the retraction state. It is needless to say that the size is not limited thereto, and the size of the angle of view may be more decreased in the insertion state than in the retraction state.

The light guiding optical system is formed on the measurement light path. The light guiding optical system includes at least an optical scanning unit (optical scanner), and may further include an objective optical system. The optical scanner deflects the measurement light from the light splitter, and thereby the light guiding optical system forms a pivot point, at which the measurement light turns based on motion of the optical scanner, in an anterior portion of a subject eye and guides the measurement light passed through the pivot point to the fundus. Scanning of the measurement light about the pivot point on the fundus is performed in accordance with the motion of the optical scanner.

The objective optical system is an optical system that is disposed between the optical scanner of the light guiding optical system and the subject eye and is used for forming the pivot point. The pivot point is formed at a conjugated position of the optical scanner with respect to the objective optical system. The pivot point is also referred to as a "first pivot point". The objective optical system may be a refraction system including a lens, a reflection system including a mirror, or a combination of both of the systems.

The angle-of-view switching optical system is inserted into and removed from the light guiding optical system on the measurement light path. In this specification, a state in which the angle-of-view switching optical system is inserted into the light guiding optical system is referred to as an "insertion state", and a state in which the angle-of-view switching optical system is retracted from the light guiding optical system is referred to as a "retraction state". The size of the angle of view, which indicates a scanning range of the measurement light on the fundus, in the insertion state may be different from that in the retraction state. For convenience of description, unless otherwise noted, the angle-of-view switching optical system includes at least one lens in the following description.

Hereinafter, a state in which the angle of view is increased in the insertion state in which the angle-of-view switching optical system is inserted into the light guiding optical system is described. In this case, in the insertion state of the angle-of-view switching optical system, the angle of view is increased from the retraction state such that the measurement light is guided to the wide-angle region of the fundus.

The angle-of-view switching optical system may be inserted and may be removed from between the objective optical system included in the light guiding optical system and the subject eye. In this case, it is more preferable to have a lens disposition of a lens position of the angle-of-view switching optical system, in which a lens having principal power in the angle-of-view switching optical system is disposed to be inserted and removed from between the first pivot point and the subject eye. In the case of the disposition, it is easy to secure a longer operation distance than that in a case where the lens having the principal power is disposed between the objective optical system and the first pivot point.

In a case where the lens having the principal power in the angle-of-view switching optical system is inserted between the first pivot point and the subject eye, the angle-of-view switching optical system relays the first pivot point in the insertion state and forms a second pivot point. Specifically, the angle-of-view switching optical system forms the second pivot point by turning the measurement light passed through the first pivot point toward an optical axis in the insertion state. In the insertion state, the second pivot point is positioned in an anterior portion of the subject eye, and thereby scanning of the measurement light is performed on the fundus.

In this disclosure, a size of the scanning range of the measurement light on the fundus is described as an "angle of view". Here, the "angle of view" more depends on performance of an optical system that is disposed on the subject eye than the optical scanner and is a value obtained by assuming that the optical scanner is appropriately operated such that a maximum angle of view is realized.

### <Compensation for Change in Light Path Length Difference>

When the angle-of-view switching optical system is inserted into and removed from the light guiding optical system, a light path length of the measurement light path is changed, and thus there can be found a light path length difference between the reference light and the measurement light. For example, since the angle-of-view switching optical system, which relays the pivot point formed by the objective optical system, is likely to be large in size and is likely to have a long light path length, the change in the light path length difference according to the insertion and the retraction is also considered to be increased. For example, there has been known a technology in which an attachment optical system is installed on an inspection window of fundus imaging OCT such that it is possible to image an anterior portion (for example, see "JP-A-2011-147612" or the like by the present applicant). In such an apparatus, a light path length of the measurement light path is changed before and after the attachment optical system is attached and detached. However, in the apparatus described above, the light path length of the attachment optical system is short, and an imaged site is switched to the anterior portion. In this manner, the light path length of the measurement light path in an ocular bulb is short, and thus a sufficient compensation length in the apparatus was about an ocular axial length (≅ 32 mm). By comparison, the light path length of the angle-of-view switching optical system in the embodiment is longer than the ocular axial length. For example, in the embodiment, it is necessary to compensate for an amount of change by about three to eight times an equivalent length to the ocular axial length in some cases. For example, in an example of design of the angle-of-view switching optical system that switches an angle of view from about 60° to about 100° in an OCT apparatus that is capable of performing imaging at the angle of view of about 60°, the light path length of the angle-of-view switching optical system was about 170 mm. In this manner, there is no configuration in the related art which is capable of coping with a change in the light path length of the measurement light path according to the insertion and the retraction of the angle-of-view switching optical system.

The OCT optical system may include a compensation unit that compensates for an amount of change in the light path length of the measurement light path between the insertion state and the retraction state.

The OCT optical system may include a plurality of reference light paths as the compensation unit. For example, the reference light path may branch to at least two paths of a first branched light path and a second branched light path. Here, the first branched light path has a first light path length corresponding to a light path length of the measurement light path in the retraction state. In addition, the second branched light path has a second light path length corresponding to a light path length of the measurement light path in the insertion state. A light path length difference between the first branched light path and the second branched light path may be determined in advance. Specifically, the light path length difference may be a length that is substantially equal to the light path length of the angle-of-view switching optical system.

The OCT optical system may cause the detector to simultaneously detect both of the interference signal due to the reference light from the first branched light path and an interference signal due to the reference light from the second branched light path or selectively detect any one of the two interference signals.

The reference lights from the light splitter are simultaneously guided to the first branched light path and the second branched light path, and thereby the interference signals due to the reference lights from both of the light paths of the first branched light path and the second branched light path can be simultaneously detected by the detector. However, in this case, a light path length difference between the measurement light and the reference light through the first branched light path and a light path length difference between the measurement light and the reference light through the second branched light path are replaced between "substantially 0" and "substantially a light path length of the angle-of-view switching optical system" based on the insertion and retraction of the angle-of-view switching optical system. Therefore, strength of one interference signal corresponding to a state (insertion state/retraction state) of the light guiding optical system of the two types of interference signals having a different path of the reference light path from each other is obviously stronger than that of the remaining one. In a case where the light path length of the angle-of-view switching optical system is sufficiently long, the interference signal of the remaining one has a level of strength which does not cause a problem. In the retraction state, the interference signal by the reference light through the first branched light path is a signal having a signal strength that is stronger than that of the interference signal by the reference light through the second branched light path, and vice versa in the insertion state. The light from the OCT light source is split to the measurement light and the reference light by the light splitter; however, it is not essential for a side of the reference light path to have an element such as the subject eye, which significantly attenuates a beam. Therefore, even when the first branched light path and the second branched light path which branch from the reference light path are formed, sufficient intensity is secured to obtain the interference signal on both of the branched light paths. Therefore, even in a configuration in which either light path length of the measurement light path or the reference light path is switched according to the insertion and the retraction of the angle-of-view switching optical system, it is possible to perform good acquisition of the OCT data based on the interference signal detected by the detector.

The OCT optical system may have, as a part of the compensation unit, a switch (an example of a drive unit) that switches the light path, through which the reference light is guided from the light splitter, between the first branched light path and the second branched light path. Consequently, the detector can selectively detect one of the interference signal by the reference light from the first branched light path and the interference signal by the reference light from the second branched light path. For example, a controller of the OCT apparatus may cause the switch to perform switching depending on the state (insertion state/retraction state) of the light guiding optical system. In other words, the switch may be controlled to be driven such that the reference light from the light splitter is guided to the first branched light path in the retraction state, and the reference light from the light splitter is guided to the second branched light path in the insertion state. A method of compensating for an amount of change in the light path length on the measurement light path is considered to be particularly advantageous in a case where the light path length of the angle-of-view switching optical system is relatively short or the like, compared with a system without the switch.

In addition, the reference light path branching as described above is not absolutely necessary to be provided. In this case, the compensation unit may adjust at least one length of the measurement light path and the reference light path, for example. For example, in a case where the compensation unit adjusts the light path length of the reference light path, the compensation unit may switch the light path length of the reference light path, to which the reference light is guided from the light splitter, between the first light path length corresponding to the light path length of the measurement light path in the retraction state and the second light path length corresponding to the light path length of the measurement light path in the insertion state. In addition, in a case where the compensation unit adjusts the light path length of the measurement light path, the compensation unit may change a light path length between the light splitter and the optical scanner on the measurement light path, thereby compensating for an amount of change in the light path length according to the insertion and the retraction of the angle-of-view switching optical system described above.

### <Dispersion Correction>

In addition, the OCT apparatus of the embodiment includes a dispersion correcting unit (dispersion compensating unit) that corrects (compensates for) a dispersion amount in an optical system between the measurement light path and the reference light path, thereby being capable of obtaining good OCT data. The dispersion correcting unit may correct the dispersion amount optically or may correct in a signal processing manner (including at least one of signal processing or calculation). In a case of the former, the dispersion correcting unit is an element of the OCT optical system. In a case of the latter, the dispersion correcting unit is an electronic circuit (may be a dedicated circuit or an image processor) which is connected to the detector.

Incidentally, in the embodiment, the angle-of-view switching optical system is inserted into or removed from the light guiding optical system, and thereby the dispersion amount in the optical system between the measurement light path and the reference light path is likely to be changed. In the OCT apparatus of the embodiment, a plurality of different correction values of the dispersion amount may be set in the dispersion correcting unit. At least a first correction value for correcting a dispersion amount in the retraction state and a second correction value for correcting the dispersion amount in the insertion state are set, and thereby it is possible to acquire good OCT data in both cases where the angle-of-view switching optical system is inserted and removed.

In addition, the correction value of the dispersion correcting unit may be further subdivided. For example, since the dispersion amount in the optical system between the measurement light path and the reference light path can vary for each scanning angle of the optical scanner, the first correction value, the second correction value, or both correction values may be set as different values for each scanning angle of the optical scanner. In particular, in a case where a scanning range is performed at a wide angle in the insertion state of the angle-of-view switching optical system, a case of having a significant difference between a dispersion amount related to measurement light, with which the fundus center area is irradiated, and a dispersion amount related to measurement light, with which the fundus peripheral area is irradiated, is considered. In the OCT apparatus of the embodiment, a different correction value may be set for each of at least the fundus center area and the fundus peripheral area.

### <Light-flux Diameter Changer>

In addition, in the OCT apparatus of the embodiment, the angle-of-view switching optical system is inserted into the light guiding optical system, and thereby a light-flux diameter of the measurement light is considered to be increased. In this case, a spot size of the measurement light on the fundus increases as a light-flux diameter increases in the insertion state, and thereby image resolving power is considered to be more degraded, compared with the retraction state. In this respect, the OCT apparatus of the embodiment includes a light-flux diameter changer. Here, the light-flux diameter changer adjusts a light-flux diameter of the measurement light with which the subject eye is irradiated. The controller (processor) of the OCT apparatus causes the light-flux diameter changer to switch the light-flux diameter in association with a state (insertion state/retraction state) of the light guiding optical system, and thereby it is possible to change the image resolving power before and after the angle-of-view switching optical system is inserted and removed. More specifically, in a case where the state is switched from the retraction state to the insertion state, the controller drives the light-flux diameter changer such that the light-flux diameter of the measurement light decreases. In addition, in a case where the state is switched from the insertion state to the retraction state, the light-flux diameter changer is driven such that the light-flux diameter of the measurement light increases. Consequently, the change in spot size of the measurement light is corrected. Since the spot size is substantially proportional to an angle of view, it is preferable to drive the light-flux diameter changer by a correction amount in association with a ratio of the angle of view (scanning range) between the insertion state and the retraction state.

### <Example>

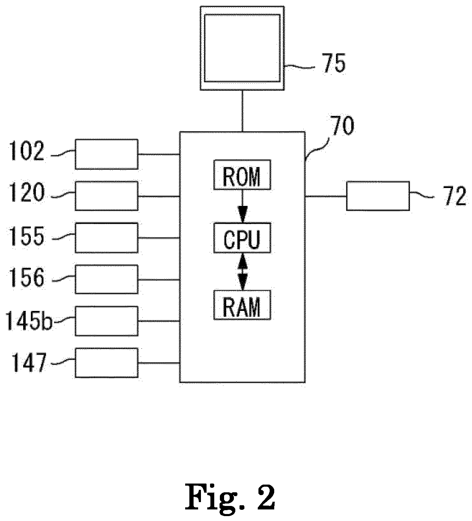

Hereinafter, an optical coherence tomography (OCT) apparatus shown in Figs. 1 and 2 will be described as an example. For example, the OCT apparatus according to the example has spectral domain OCT (SD-OCT) as a basic configuration.

An OCT apparatus 1 includes a light source 102, an interference optical system (OCT optical system) 100, and a calculation controller (calculation control unit) 70 (refer to Fig. 2). Additionally, in the OCT apparatus, a memory 72, a display unit 75, a front image observing system and a fixation target projecting system (not shown) may further be provided. The calculation controller (hereinafter, a controller) 70 is connected to the light source 102, the interference optical system 100, the memory 72, and the display unit 75.

The interference optical system 100 guides the measurement light to an eye E by a light guiding optical system 150. The interference optical system 100 guides the reference light to a reference optical system 110. The interference optical system 100 causes a detector (light receiving element) 120 to receive an interference signal light that is acquired due to interference of the reference light with the measurement light reflected from the eye E. The interference optical system 100 is mounted in a housing (apparatus main body) (not shown), and the housing moves three-dimensionally with respect to the eye E by a known alignment moving mechanism via an operation unit such as a joystick. In this manner, alignment may be performed with respect to the subject eye.

The SD-OCT type may be used for the interference optical system 100. A light source that emits a light-flux having a short coherence length is used as the light source 102, and a spectroscopic detector that performs spectroscopic dispersion and detects a spectral interference signal for each wavelength component is used as the detector 120.

A coupler (splitter) 104 is used as a first light splitter and splits the light emitted from the light source 102 to light traveling to the measurement light path and light traveling to the reference light path. For example, the coupler (fiber coupler) 104 guides the light from the light source 102 to an optical fiber 152 on a side of the measurement light path and guides the light to a coupler (fiber coupler) 140 on a side of the reference light path via a fiber 106. Consequently, the light from the light source 102 is guided to the reference optical system 110.

### <Light Guiding Optical System>

The light guiding optical system 150 is provided to guide the measurement light to the eye E. For example, in the light guiding optical system 150, the optical fiber 152, a collimator lens 154, a variable beam expander 155, an optical scanner 156, and an objective lens system 158 (the objective optical system in the example) are provided in this order. In this case, the measurement light is emitted from an emission end of the optical fiber 152 and becomes a parallel beam by the collimator lens 154. Then, the light travels toward the optical scanner 156 in a state of having a desired light-flux diameter by the variable beam expander 155. The eye E is irradiated with the beam passing through the optical scanner 156 via the objective lens system 158. A first pivot point P1 is formed at a conjugated position of the optical scanner 156 with respect to the objective lens system 158. The anterior portion is positioned at the pivot point P1, and thereby the measurement light reaches the fundus without eclipse. In addition, the fundus is scanned with the measurement light depending on the operation of the optical scanner 156. In this case, the measurement light is scattered-reflected by tissue of the fundus.

The optical scanner 156 may scan the eye E with the measurement light in XY directions (transverse directions). For example, the optical scanner 156 is configured of two galvano mirrors, and a reflection angle of the mirror is adjusted optionally by a driving mechanism. A reflection (traveling) direction of the light-flux emitted from the light source 102 changes, and the fundus is scanned in any optional direction. For example, as the optical scanner 156, an acousto-optic modulator (AOM) or the like that changes the traveling (deflection) direction of light may be used, in addition to a reflective mirror (a galvano mirror, a polygon mirror, or a resonant scanner).

Scattered light (reflected light) of the measurement light from the eye E travels back via a path used during light projection, is incident to the optical fiber 152, and reaches the coupler 104. The coupler 104 guides the light from the optical fiber 152 to a light path toward the detector 120.

### <Attachment Optical System>

An attachment optical system 160 (an example of the "angle-of-view switching optical system") in the OCT apparatus according to the example is inserted and removed from between the objective optical system 158 in the light guiding optical system 150 and the subject eye E. A lens tube including the attachment optical system is attached to and detached from a housing surface (not shown), and thereby the attachment optical system 160 is inserted and removed from between the objective optical system 158 and the subject eye E.

The attachment optical system 160 may include a plurality of lenses 161 to 164. Here, a lens having principal power in the attachment optical system 160 shown in Fig. 1 is the lens 164 placed in front of the subject eye. An insertion/retraction position of at least the lens 164 is between the first pivot point P1 that is formed by the objective optical system 158 and the subject eye E. At least the lens 164 turns the measurement light passed through the first pivot point P1 toward an optical axis L, and thereby a second pivot point P2 is formed at a conjugated position of the optical scanner 156 with regard to the attachment optical system 160 the objective optical system 158. In other words, the attachment optical system 160 is an optical system that relays the pivot point P1 to the pivot point P2.

In the example, a solid angle of the measurement light at the second pivot point P2 is larger than a solid angle at the first pivot point P1. For example, the solid angle at the second pivot point P2 is increased as twice or more as the solid angle at the first pivot point P1. In the example, it is possible to perform scanning at an angle of view of about 60° in the retraction state, and it is possible to perform scanning at an angle of view of about 100° in the insertion state.

The variable beam expander 155 is the light-flux diameter adjusting unit in the example. As an example, the variable beam expander 155 may include a plurality of lenses that form a both-side telecentric optical system and may be configured to switch a light-flux diameter by changing a lens space by an actuator. The variable beam expander 155 adjusts the light-flux diameter of the measurement light based on an instruction from the controller 70.

If the light-flux diameter of the measurement light that is guided from the variable beam expander 155 to the optical scanner 156 is constant between the insertion state and the retraction state, the spot size of the measurement light is proportional to the angle of view on the fundus. Therefore, the resolving power is more degraded in the insertion state than in the retraction state. In the example, the controller 70 drives the variable beam expander 155 according to the insertion and the retraction of the attachment optical system and more decreases the light-flux diameter in the insertion state than in the retraction state. A rate of the light-flux diameters (light-flux diameters in the variable beam expander 155) in the insertion state and the retraction state is inversely proportional to the angle of view in the insertion state and the retraction state, and thereby it is possible to suppress a change in resolving power based on the insertion and retraction of the attachment optical system 160.

Incidentally, in order to secure a sufficient operation distance, the attachment optical system 160 needs to cause the measurement light to be tuned from a position having a sufficient light beam height toward the optical axis L. In addition, in order to suppress an aberration caused by the attachment optical system 160 within a permissible range, power of the lenses included in the attachment optical system 160 is limited. Hence, it is difficult to shorten the light path length of the attachment optical system 160.

Although there is an OCT apparatus in the related art that is configured to adjust the light path length difference between the reference light and the measurement light, there is no OCT apparatus that has an adjustment range that is applicable to the insertion and retraction of the attachment optical system 160. For example, in the related art, there has been known a technology in which an optical adapter is installed such that it is possible to perform imaging an anterior portion in fundus imaging OCT (for example, see "JP-A-2011-147612" or the like by the present applicant). However, the optical adapter does not relay the pivot point formed by an optical system of an apparatus main body, and there is no demand for a wide-angle scanning range. Therefore, the optical adapter can be formed to have a relatively short light path length. Further, a position of an image surface is changed from the fundus to the anterior portion in response to insertion of the optical adapter. Hence, there is no need to significantly adjust the light path length difference in response to the insertion of the optical adapter.

### <Reference Optical System>

The reference optical system 110 generates the reference light that is combined with fundus reflection light of the measurement light. The reference light passing through the reference optical system 110 is coupled and interferes with light from the measurement light path by a coupler (Fiber coupler) 148. The reference optical system 110 may be one of a Michelson type or a Mach-Zehnder type.

The reference optical system 110 shown in Fig. 1 is formed by a transmission optical system. In this case, the reference optical system 110 does not cause the light from the coupler 104 to return but transmits the light, thereby guiding the light to the detector 120. The reference optical system 110 is not limited thereto and may be formed by a reflection optical system and guide the light from the coupler 104 to the detector 120 by causing the light to be reflected from the reflection optical system, for example.

In the example, the reference optical system 110 may have a plurality of reference light paths. For example, in Fig. 1, the coupler (fiber coupler) 140 causes the reference light path to branch to a light path (the first branched light path in the example) via which a fiber 141 passes and a light path (the second branched light path in the example) via which a fiber 142 passes. The fiber 141 and the fiber 142 are connected to a coupler (fiber coupler) 143. Consequently, two branched light paths are coupled, and light is incident to the coupler 148 via a light path length difference adjusting unit 145 and a polarization controller 147.

In the example, the reference light from the coupler 104 is simultaneously guided to the fiber 141 and the fiber 142 by the coupler 143. Light passing through either the fiber 141 or the fiber 142 is coupled with the measurement light (fundus reflection light) in the coupler 148.

A light path length difference between the fiber 141 and the fiber 142, that is, a light path length difference between the first branched light path and the second branched light path, may be a fixed value. In the example, the light path length difference is set to be substantially equal to the light path length of the attachment optical system 160.

An optical member for adjusting the light path length difference between the measurement light and the reference light may be disposed on at least any one of the measurement light path and the reference light path. As an example, the reference light path adjusting unit 145 is provided in the optical system shown in Fig. 1, and a mirror 145a having two orthogonal surfaces is provided at a corresponding position so as to control the light path length difference between the measurement light and the reference light. The mirror 145a moves in an arrow direction by an actuator 145b, and thereby it is possible to increase and decrease the light path length of the reference light path. It is needless to say that a configuration, in which the light path length difference between the measurement light and the reference light is adjusted, is not limited thereto. For example, in the light guiding optical system 150, the collimator lens 154 and a coupler 153 move integrally, and thereby the light path length of the measurement light is adjusted. As a result, the light path length difference between the measurement light and the reference light may be adjusted.

Here, In the example, since the reference light path adjusting unit 145 is provided on a light path between the coupler 143 and the coupler 148, that is, on a common light path between the first branched light path and the second branched light path, it is possible to perform adjustment of an ocular axial length with regard to an individual difference, which is the adjustment of the light path length difference between the measurement light path and the reference light path, with respect to both of the first branched light path and the second branched light path.

It is preferable that an adjustment range of the light path length in the reference light path adjusting unit 145 is set to be sufficiently shorter than the light path length difference between the fiber 141 and the fiber 142 (in other words, the light path length difference between the first branched light path and the second branched light path).

### <Correction of Manufacturing Tolerance of Zero-delay Position>

Incidentally, a zero-delay position (a position at which the light path length difference between the measurement light path and the reference light path is zero) is considered to be different for each device of the attachment optical system 160 due to a manufacturing tolerance of the attachment optical system 160 and the fiber 142. For example, the manufacturing tolerance can be corrected by adjusting an initial distance value of a distance between the collimating lens 154 and a diopter correcting lens in the light guiding optical system 150. The OCT apparatus 1 may include an adjusting mechanism (not shown) for adjusting such a manufacturing error. The adjusting mechanism may be configured to be capable of performing adjustment after a product delivery of the OCT apparatus 1.

### <Light Detector>

The detector 120 is provided to detect interference of the light from the measurement light path with the light from the reference light path. In the example, the detector 120 is a spectroscopic detector and includes an optical spectrometer and a line sensor, for example, in which the measurement light and the reference light which are coupled by the coupler 148 are scattered by the optical spectrometer and are received in a different region (pixel) of the line sensor for each wavelength. Consequently, an output for each pixel is acquired as a spectral interference signal.

Since a curvature of the fundus does not necessarily match an image forming surface of the measurement light, and a displacement between both of a fundus center area and a fundus peripheral area increases in at least one of the areas in the insertion state of the attachment optical system 150, it is preferable to secure a sufficient depth range in the light detector in consideration of the displacement. For example, in the SD-OCT, it is preferable to provide a line camera having a sufficient number of pixels with respect to an anticipated depth range. In addition, the following configuration may be further employed as "modification examples".

### <Acquisition of Depth Information>

The controller 70 performs processing (Fourier analysis) on a spectral signal detected by the detector 120 and obtains OCT data of the subject eye.

The spectral signal (spectral data) may be rewritten as a function of a wavelength λ and may be converted to an equal interval function I (k) with regard to a wave number k (= 2 π /λ). Alternatively, the spectral signal may be acquired as an equal interval function I (k) with regard to the wave number k from the beginning (a k-clock technology). The calculation controller may perform Fourier transform of the spectral signal in a space having the wave number k, thereby obtaining OCT data in a depth (Z) region.

Further, information after the Fourier transform may be obtained as a signal containing a real component and an imaginary component in a Z space. The controller 70 may obtain absolute values of the real component and the imaginary component in the signal in the Z space, thereby acquiring the OCT data.

Here, in the coupler 148, the reference light passing through the first branched light path and the reference light passing through the second branched light path are simultaneously guided and each is coupled to the measurement light. Since a large light path length difference, which is substantially equal to the light path length of the attachment optical system 160, occurs between the first branched light path and the second branched light path, one of the reference light passing through the first branched light path and the reference light passing through the second branched light path is likely to interfere with the measurement light; however, the other reference light is unlikely to interfere with the measurement light. Although the spectral interference signal from the detector 120 contains a component due to the reference light passing through the first branched light path and a component due to the reference light passing through the second branched light path, one of the two types of components according to the state of the light guiding optical system 150 is obtained as a remarkably stronger signal than the other. As a result, it is possible to obtain good OCT data regardless of the state of the light guiding optical system 150. In other words, the OCT apparatus according to the example has the light path length difference corresponding to the attachment optical system 160 and includes the plurality of reference light paths, and thereby the amount of change according to the insertion and the retraction of the attachment optical system 160, which is the amount of change in the light path length difference on the measurement light path, is compensated regardless of the state of the light guiding optical system 150.

The reference light path adjusting unit 145 needs to be controlled to adjust the light path length difference related to the ocular axial length of the subject eye E, which is the light path length difference between the measurement light path and the reference light path, in advance. For example, in the example, the mirror 145a may be moved in a predetermined adjustment range, an interference signal may be acquired at each position, and the position of the mirror 145a may be determined on the basis of a position, at which the strength of the interference signal has the highest strength. In a case where the adjustment range of the light path length in the reference light path adjusting unit 145 is sufficiently small with respect to the light path length difference between the first branched light path and the second branched light path, at a position in the adjustment range of the reference light path adjusting unit 145, at which the interference signal has a strength peak, can be uniquely identified.

Since the fundus reflection light of the measurement light from the fundus peripheral area is weaker than the reflected light from the fundus center area in the insertion state, the light path length difference between the measurement light path and the reference light path may be adjusted by the reference light path adjusting unit 145 such that the zero-delay position between the measurement light path and the reference light path overlaps anticipated fundus tissue (for example, the retina, the choroid, the sclera, or the like) in the fundus peripheral area.

### <Dispersion Correction by Software>

In the present embodiment, a controller 70 may perform the dispersion correction processing on the spectrum data output from the detector 120 using software. The controller 70 obtains the OCT data based on the spectrum data after the dispersion correction. Therefore, there occurs a difference in image quality between the real image and the virtual image.

That is, in the present embodiment, a difference in the amount of dispersion of the optical system between a measurement light path and a reference light path is corrected by signal processing. Specifically, the correction value stored in advance in the memory 72 is applied in the spectrum signal processing.

The controller 70 acquires a spectrum intensity of the light based on the received light signal output from the detector 120 and rewrites the spectrum intensity as a function of the wavelength λ. Next, the spectrum intensity I (λ) is converted into a function I (k) that is evenly spaced with respect to the wave number k (= 2π/λ).

The influence of the dispersion mismatch between the measurement light and the reference light shifts the phase of the interference component, decreases the peak of the multiplexed signal of each wavelength, and spreads the signal (decreases the resolution). Therefore, in the dispersion correction, by returning the phase shifted for each wavelength, the decrease in the resolution due to the decrease in the interference signal is corrected. In this case, the phase shift amount φ (k) as a function of the wave number k is obtained, and the phase shift is returned for each value of k by "I (k) × exp-iφ (k)". Here, the phase φ (k) to be subjected to the dispersion correction can be obtained in advance by calibration, or the phase φ (k) corresponding to the acquired tomographic image may be obtained. Then, a parameter for dispersion correction (for example, a phase φ (k)) is stored in the memory 72.

Thereafter, the controller 70 obtains the OCT data by performing the Fourier transform on the spectrum intensity I (k) after the dispersion correction which is a result of correction by the set dispersion correction data.

For example, a first dispersion correction value (for normal image) is acquired from the memory 72 as a dispersion correction value for correcting the influence of the dispersion on the real image, the spectrum data output from the detector 120 is corrected using the first dispersion correction value, and the Fourier transform is performed on the corrected spectral intensity data to form the OCT data. The real image R is acquired as a high sensitivity and high resolution image, and the virtual image M (the mirror image) is acquired as a low resolution blurred image due to the difference in dispersion correction value.

In this way, when a real image R is acquired in a first image area G1, the real image R is acquired as a high sensitivity and high resolution image, and the virtual image M (the mirror image) is acquired in a second image area G2 as a low resolution blurred image due to the difference in dispersion correction value. On the other hand, when a real image R is acquired in the second image area G2, the virtual image M is acquired in the first imaging area G1 as a low resolution blurred image due to the difference in dispersion correction value.

Of course, not limited to the description above, the software dispersion correction may be performed on the virtual image M. In this case, the virtual image M is acquired as a high sensitivity and high resolution image, and the real image R is acquired as a low resolution blurred image.

For details of the method of performing the dispersion correction by the software described above, US Patent No. 6980299 and JP-T-2008-501118, and the like may be referred to. In addition, JP-A-2010-29648 may be referred to.

In a case where the dispersion correction processing by the software is performed, when obtaining the OCT data on the fundus center area, for example, the controller 70 may extract the image data having the higher sensitivity and resolution among the image data of the real image and the virtual image.

In the example, a first correction value corresponding to the retraction state and a second correction value which is a value different from the first correction value and corresponds to the insertion state are stored in a memory 72 in advance, and a correction value to be applied is switched according to the state of the light guiding optical system 150. As a result, in the OCT apparatus according to the example, an amount of change according to the insertion and retraction of the attachment optical system 160, which is an amount of change in the dispersion amount between the measurement light path and the reference light path, is compensated in each state of the light guiding optical system 150.

Further, in the example, a plurality of second correction values corresponding to the insertion state are set depending on scanning positions of the measurement light. Specifically, a correction value for the fundus center area and a correction value for the fundus peripheral area are set as second correction values which are different from each other. For example, the first correction value may be applied to a region within an angle of 60° of the fundus, and the second correction value may be set as a value that is applied to a region apart from the region at the angle of 60°. Since the attachment optical system 160 has significant power, overall, a significant difference in dispersion amount is considered to occur between a light-flux passing through the fundus center area and a light-flux passing through the fundus peripheral area. By comparison, in the example, since the correction value of the dispersion amount changes depending on an irradiation position of the fundus with the measurement light, it is possible to obtain good OCT data in the wide-angle region of the fundus.

It is needless to say that the second correction value may be further subdivided. For example, the entire fundus is divided into the fundus center area, a first fundus peripheral area on an outer side from the fundus center area, and a second fundus peripheral area on an outer side from the first fundus peripheral area, and a correction value corresponding to the fundus center area, a correction value corresponding to the first fundus peripheral area, and a correction value corresponding to the second fundus peripheral area may be set to be different from each other as the second correction value.

### <Control System>

The controller 70 may include a CPU (processor), a RAM, a ROM, and the like (refer to Fig. 2). For example, the CPU of the controller 70 may control the OCT apparatus. The RAM stores various types of information temporarily. For example, in the ROM of the controller 70, various programs for controlling operations of the OCT apparatus, the initial value, or the like may be stored.

The non-volatile memory (hereinafter, simply abbreviated to "memory") 72 as the storage unit, the display unit 75, and the like are electrically connected to the controller 70. A non-transitory storage medium, which is capable of storing the storage content even when an electric power supply is cut off, may be used for the memory 72. For example, a hard disk drive, a flash ROM, a USB memory that is removably installed in the OCT apparatus or the like can be used as the memory 72. The memory 72 may store a control program for controlling the acquisition of the OCT data and the imaging of an OCT image. In addition, the memory 72 may store various types of information related to imaging other than the OCT image generated from the OCT data. The display unit 75 may display the OCT image generated from the OCT data.

An insertion/retraction detector that automatically detects whether or not the attachment optical system 160 is inserted into the light guiding optical system may be provided, and the controller may perform control or processing of each member of the OCT optical system 100 based on a detection signal from the detector. For example, switching control of the light-flux diameter by the variable beam expander 155, setting control of the zero-delay position by the reference light path adjusting unit 145, change processing of the dispersion amount in the optical system between the measurement light path and the reference light path, or the like may be appropriately performed as described above. The insertion detector may be a sensor disposed in the vicinity of the objective optical system 158.

It is needless to say that an examiner may input information that identifies the state of the light guiding optical system (the insertion state/retraction state of the attachment optical system) on a user interface (UI) of the OCT apparatus, and thereby the controller may perform the control and the processing of each member in the OCT optical system 100 based on the corresponding information.

### <Imaging Mode Setting>

In the OCT apparatus according to the present embodiment, the first imaging mode for obtaining the OCT data on the fundus center area and the second imaging mode for obtaining the OCT data on the wide-angle region including the fundus center area and the fundus peripheral area may be settable. In this case, for example, in the first imaging mode, the scanning range of the measurement light on the fundus may be set on the fundus center area, and in the second imaging mode, the scanning range of the measurement light onto the fundus may be set on the wide-angle region including the fundus center area and the fundus peripheral area.

In this case, for example, the controller 70 may switch the imaging mode between the first imaging mode and the second imaging mode based on an operation signal from an operation unit operated by the examiner. In addition, the controller 70 may automatically switch the imaging mode between the first imaging mode and the second imaging mode. In addition, the controller 70 may perform a guide display prompting the switching of the imaging mode between the first imaging mode and the second imaging mode.

The controller 70 may automatically switch the imaging mode between the first imaging mode and the second imaging mode based on a detection signal from an insertion and retraction detection unit (may perform the guide display). If the scanning range of the measurement light is within the predetermined range, the controller 70 may set the imaging mode as the first imaging mode, and if the scanning range of the measurement light exceeds the predetermined range, then, may set the imaging mode as the second imaging mode. The mode switching may be performed automatically or via the guide display.

After automatically adjusting a difference in optical path length between the measurement light and the reference light, if the real image R of the OCT data in the first image area G1 is on the zero delay (or if the real image R of the OCT data is detected in the second image area G2 also), the controller 70 may automatically switch the imaging mode to the second imaging mode or may perform the guide display. If the difference in optical path length is automatically adjusted, an adjustment may be performed such that a retinal portion in the OCT data of the fundus center area is formed in the first image area G1.

### <Switching of Display State of OCT Data according to Imaging Mode>

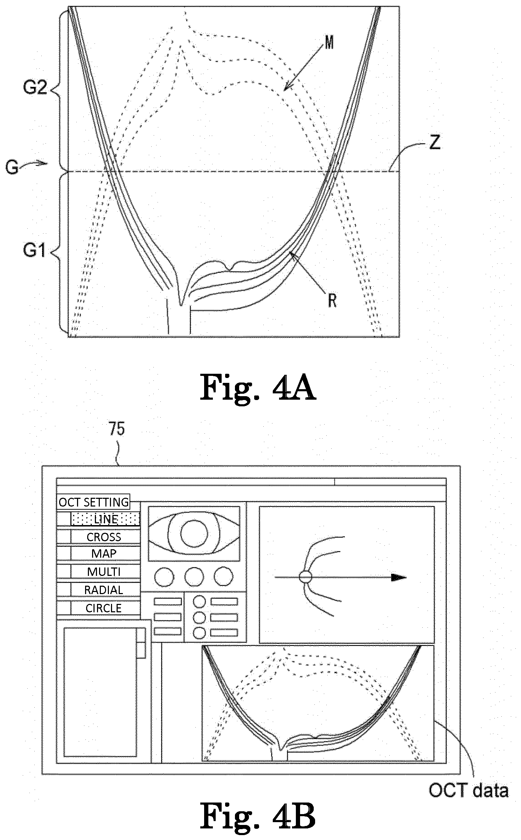

The controller 70 may switch the display state of the OCT data on the display unit 75 between the first imaging mode and the second imaging mode (for example, refer to Figs. 3A, 3B, 4A and 4B). In this case, for example, the controller 70 may change the output range of the OCT data on a display screen of the display unit 75 according to the imaging mode. If the imaging mode is set as the first imaging mode, the controller 70 may set the output range of the OCT data in the depth direction as a first output range, and if the imaging mode is set as the second imaging mode, the controller 70 may set the output range of the OCT data in the depth direction as a second output range which is wider than the first output range. In this case, in the second the output range, the output range may be set such that OCT data of an area from the fundus center area to the fundus peripheral area is output to the display screen of the display unit 75.

The controller 70 may change the display magnification of the OCT data on the display screen of the display unit 75 according to the imaging mode. In this case, the controller 70 may change the vertical magnification, or may change at least one of the vertical magnification and the horizontal magnification. In addition, the controller 70 may change the display range of the OCT data on the display screen of the display unit 75. In this case, the controller 70 may change the display range in the vertical direction, or may change the display range in at least one of the vertical direction and the horizontal direction.

Figs. 3A and 4A are diagrams illustrating examples of an image G shown by the OCT data acquired by the OCT optical system 200, and Figs. 3B and 4B are diagrams illustrating examples of the output on the display unit. A zero delay position Z on the image G shown by the OCT data is a position of the reference light in the OCT data corresponding to an optical path length and corresponds to a position where the optical path length of the measurement light and the reference light match each other. The image G shown by the OCT data is formed of a first imaging area G1 corresponding to the inner side (the rear side) of the zero delay position Z and the second imaging area G2 corresponding to the front side of the zero delay position Z. According to the invention, the first imaging area G1 and the second imaging area G2 have a symmetrical relation with respect to the zero delay position Z.

Fig. 3A is a diagram illustrating an example of an image G shown by OCT data obtained in the first imaging mode, and Fig. 3B is a diagram illustrating an example of an output of the OCT data obtained in the first imaging mode on the display unit.

For example, the controller 70 may adjust the difference in optical path length between the measurement light and the reference light such that the retinal surface in the fundus center area is formed at the inner side (the rear side) of the zero delay position Z (retinal mode) or such that the rear side of a choroid in the fundus center area is formed at the front side of the zero delay position Z (choroid mode).

When the retinal surface in the fundus center area is formed at the inner side (the rear side) of the zero delay position Z (retinal mode), each of the tomographic images formed in the first imaging area and in the second imaging area is in a state of facing each other (refer to Fig. 3A). The real image R is acquired in the first imaging area G1 and the virtual image M (mirror image) is acquired in the second imaging area G2.

Although not illustrated, when the rear side of the choroid in the fundus center area is formed at the front side of the zero delay position Z (choroid mode), each of the tomographic images formed in the first imaging area G1 and in the second imaging area G2 is in a state of facing opposite direction from each other. The virtual image M is acquired in the first imaging area G1 and the real image R is acquired in the second imaging area G2.

For example, the controller 70 may extract any one of the image data of the first imaging area G1 or of the second imaging area G2 in the OCT data, and may display an image shown by the extracted image data on the screen of the display unit 75 (refer to Fig. 3B). As a result thereof, for example, only the real image R is displayed on the display unit 75, and the virtual image M is not displayed. In this way, for example, the examiner can observe the tomographic image of the fundus center area as a single tomographic image. As the OCT data of the fundus center area, for example, the OCT data including at least one of macula and papilla may be acquired.

When the image data is extracted and displayed, for example, the controller 70 may cut the image data from the OCT data or may create new image data from information corresponding to the image data.

Fig. 4A is a diagram illustrating an example of an image G shown by OCT data acquired in the second imaging mode, and Fig. 4B is a diagram illustrating an example of the OCT data obtained in the second imaging mode output on the display unit. In this case, for example, the controller 70 may adjust the difference in optical path length between the measurement light and the reference light such that the retinal surface in the fundus center area is formed at the inner side (the rear side) of the zero delay position Z and such that the rear side of the choroid in the fundus peripheral area is formed at the front side of the zero delay position Z.

In at least a part of the OCT data of the fundus center area, each of the tomographic images formed in the first and second image areas is in a state of facing each other. The real image R is acquired in the first imaging area G1 and the virtual image M (mirror image) is acquired in the second imaging area G2.

In at least a part of the OCT data in the fundus peripheral area, each of the tomographic images formed in the first imaging area and the second imaging area are in a state of facing the opposite direction. The virtual image M is acquired in the first imaging area G1 and the real image R is acquired in the second imaging area G2.

The fundus center area and the fundus peripheral area are relative and boundaries are not clearly defined, however, at least the area including both end portions in the OCT data of the fundus peripheral area is in a state of facing the opposite direction. In this case, a portion of the OCT data of the fundus peripheral area (the fundus center area side) can be in a state of facing each other.

For example, the controller 70 may extract the image data of both the first imaging area G1 and the second imaging area G2 in the OCT data, and may display an image shown by the extracted image data on the screen of the display unit 75. As a result thereof, for example, the tomographic image of the wide-angle region including the fundus center area and the fundus peripheral area is displayed on the display unit 75. In this way, for example, the examiner can observe the tomographic image of the wide-angle region of the fundus. In this case, for example, both the real image R and the virtual image M are displayed, however, the tomographic images intersect at only a partial portion and the intersection is made around the fundus where the retinal thickness is thin, and thus, the impact on the observation is not so significant. In addition, the image quality of one image can be low sensitivity and low resolution image by the dispersion correction by the software described above, it becomes easy to observe the other image.

In the OCT data on the wide-angle region, for example, the OCT data including the macula and the papilla may be acquired as the OCT data of the fundus center area, and for example, the OCT data of a peripheral region rather than the macula and the papilla may be acquired as the OCT data of the fundus peripheral area. In this case, at least a part of the OCT data of the peripheral region is acquired as the image data in the second imaging area.

In the description above, if the display state on the display unit is switched between the first imaging mode and the second imaging mode, the controller 70 may switch the display state when displaying the OCT data with a moving image as a live image. In this case, the OCT data may be displayed together with a fundus front image, and a graphic (for example, a line) indicating the scanning range may be displayed on the fundus front image. In addition, when the OCT data is displayed as a still image after acquiring the OCT data as a capture image, the controller 70 may switch the display state.

When storing the OCT data acquired as the capture image in the memory 72, the controller 70 may store the OCT data in the memory 72 in association with the imaging mode. In this way, when viewing the OCT data with the viewer software also, the controller 70 can switch the display state according to the imaging mode. Since the display state of the display unit 75 is switched according to the imaging mode, the controller 70 may store the OCT data in association with the display state of the display unit 75 when the capture operation is performed.

### <Switching of Analysis Processing of OCT Data according to Imaging Mode>

The controller 70 may perform the analysis processing on the acquired OCT data to obtain an analysis result. For example, the controller 70 may perform segmentation processing on the OCT data and may obtain a layer thickness or a curvature of the retina or the choroid as an analysis result. In addition, if the OCT data is OCT motion contrast data (OCT angio data), analysis processing may be performed on the OCT motion contrast data to obtain the blood vessel density as an analysis result. The analysis result may be output to the display unit 75 and may be displayed, for example, as a numerical value or as an analysis map or an analysis chart.

In the analysis processing, for example, the controller 70 may change the analysis range of OCT data according to the imaging mode. When the imaging mode is set as the first imaging mode, the controller 70 may set the analysis range of the OCT data in the depth direction as the first analysis range, and when the imaging mode is set as the second imaging mode, the controller 70 may set the analysis range of the OCT data in the depth direction as the second analysis range which is wider than the first analysis range. In this case, in the second analysis range, the analysis range may be set such that the analysis processing is performed in the wide-angle region including the fundus center portion and the fundus peripheral area.

When analyzing the OCT data acquired in the first imaging mode, the controller 70 may analyze the image data in any one of the first imaging area G1 or the second imaging area G2 in the OCT data and may acquire the analysis result. As a result thereof, for example, the analysis result of the fundus center area is obtained. As a analysis result of the fundus center area, for example, an analysis result on at least one of the macula and the papilla may be obtained.

When analyzing the OCT data acquired in the second imaging mode, the controller 70 may analyze the image data in both the first imaging area G1 and the second imaging area G2 in the OCT data and may acquire the analysis result. In this case, at least the image data of the fundus center area obtained in the first imaging area G1 may be analyzed and the image data of the fundus peripheral area obtained in the second imaging area G2 may be analyzed. As a result thereof, for example, the analysis result of the wide-angle region including the fundus center area and the fundus peripheral area is obtained. As the analysis result of wide-angle region, the analysis result on the macula and the papilla and the analysis result on the area around the macula and the papilla may be obtained.

### <Composition of OCT Data and Analysis Result>

The controller 70 may synthesize the OCT data on the fundus center area acquired in the first imaging mode and the OCT data on the wide-angle region acquired in the second imaging mode by image processing, and may obtain a composite OCT data. The acquired composite OCT data may be displayed on the display unit 75. The controller 70 may perform position matching between the data by performing the matching processing in a common data area (for example, the OCT data of the fundus center area). In addition, a deviation of the imaging magnification between the data may be adjusted. Regarding the OCT data on the fundus center area, the OCT data obtained in the first imaging mode may be used or may be synthesized so as to be prioritized in the weighted composition.

In this case, for example, the OCT data on the fundus center area acquired in the first imaging mode can be acquired in higher density than OCT data of the fundus center area included in the OCT data on the wide-angle region acquired in the second imaging mode (for example, because the imaging time is short). Therefore, since the composite OCT data is acquired as the OCT data of the wide-angle region including the OCT data of the fundus center area with excellent resolution, for example, the examiner can observe the region including the macula or the papilla of the optical nervous system with high accuracy, and it is possible to reliably observe the fundus disease in the fundus peripheral area.

The controller 70 integrate the analysis result of the fundus center area based on the OCT data acquired in the first imaging mode and the analysis result of the fundus peripheral area based on the OCT data on the wide-angle region acquired in the second imaging mode, and may acquire an integrated analysis result. The acquired integrated analysis result may be displayed on the display unit 75. In acquiring the integrated analysis result, the controller 70 may acquire the integrated analysis result by analyzing the composite OCT data described above. In addition, the controller 70 may separately analyze the OCT data acquired in the first imaging mode and the OCT data acquired in the second imaging mode, and then, may integrate each results of analysis.

In the description above, the OCT apparatus for imaging a subject eye at a wide angle is used as an example, but is not limited thereto. The present embodiment may be applied to the OCT apparatus for imaging the OCT data of the examination target object at the wide angle. In addition, the examination target object may, for example, be a material other than a living body as well as the living body such as an eye (anterior eye portion, fundus, and the like), skin, and the like.

- 70: controller
- 100: OCT optical system
- 75: display unit

## Claims

1. An OCT apparatus (1) comprising:
an OCT optical system (100) that has a light splitter (104) splitting light from an OCT light source (102) to light travelling to a measurement light path and light travelling to a reference light path, and a detector (120) detecting a spectrum interference signal of measurement light guided onto a fundus of a subject eye (E) via the measurement light path and reference light from the reference light path; and
a control unit (70) configured to output OCT data acquired based on the spectrum interference signal to a display unit (75), and switch a display state of the display unit (75) between a first imaging mode for obtaining OCT data on a fundus center area of the subject eye (E) and a second imaging mode for obtaining OCT data on a wide-angle region including the fundus center area and a fundus peripheral area of the subject eye (E), wherein
in a case where the first imaging mode is set, the control unit (70) outputs OCT data of any one of front (G2) and rear (G1) image areas with respect to a zero delay position (Z) on an image (G) shown by OCT data to the display unit (75),
wherein the front (G2) and the rear (G1) image areas have a symmetrical relation with respect to the zero delay position (Z),
**characterised in that**
in a case where the second imaging mode is set, the control unit (70) outputs OCT data of both the front (G2) and rear (G1) image areas with respect to a zero delay position (Z) on an image (G) shown by OCT data to the display unit (75).

2. The OCT apparatus (1) according to claim 1,
wherein in a case where the control unit (70) outputs OCT data of both the front (G2) and rear (G1) image areas with respect to a zero delay position (Z) on an image (G) shown by OCT data to the display unit (75), the control unit (70) processes to remove one of a real image (R) and a virtual image (M), and afterward processes to display the other image on the display unit (75).

3. The OCT apparatus (1) according to claim 1 or 2, further comprising:
a storage unit (72) configured to store dispersion correction data for correcting dispersion in OCT data,
wherein the control unit (70) corrects the spectrum interference signal using dispersion correction data acquired from the storage unit (72), and performs Fourier analysis on the corrected spectrum interference signal to acquire OCT data.

4. The OCT apparatus (1) according to any one of claims 1 to 3, further comprising:
an analysis processing unit (70) configured to analyze the OCT data to obtain an analysis result,
wherein in a case where the analysis processing unit (70) analyzes OCT data acquired in the first imaging mode, the analysis processing unit (70) analyzes any one of front (G2) and rear (G1) image areas with respect to a zero delay position (Z) on an image (G) shown by OCT data to acquire the analysis result, and
in a case where the analysis processing unit (70) analyzes OCT data acquired in the second imaging mode, the analysis processing unit (70) analyzes both the front (G2) and rear (G1) image areas with respect to a zero delay position (Z) on an image (G) shown by OCT data to acquire the analysis result.

5. The OCT apparatus (1) according to any one of claims 1 to 4,
wherein the control unit (70) processes to display an image (G) shown by OCT data including both real image (R) and virtual image (M) on the display unit (75).

6. The OCT apparatus (1) according to any one of claims 1 to 5,
wherein the control unit (70) synthesizes OCT data on the fundus center area acquired in the first imaging mode and OCT data on the wide-angle region including the fundus center area and the fundus peripheral area acquired in the second imaging mode, and processes to display an image (G) shown by composite OCT data on the display unit (75).

7. The OCT apparatus (1) according to any one of claims 1 to 6,
wherein the control unit (70) switches the display state of the display unit (75) according to switching of an imaging mode between the first imaging mode and the second imaging mode.

8. The OCT apparatus (1) according to any one of claims 1 to 7,
wherein the control unit (70) switches the display state of the display unit (75) according to switching of an imaging mode by insertion and retraction of a wide angle attachment (160) to and from the OCT optical system (100).

9. The OCT apparatus (1) according to any one of claims 1 to 8,
wherein the control unit (70) changes an output range of OCT data on a display screen of the display unit (75) according to switching of an imaging mode between the first imaging mode and the second imaging mode.

10. The OCT apparatus (1) according to any one of claims 1 to 9,
wherein in a case where the first imaging mode is set, the control unit (70) sets an output range of OCT data in a depth direction as a first output range, and
in a case where the second imaging mode is set, the control unit (70) sets the output range of OCT data in the depth direction as a second output range which is wider than the first output range.

11. The OCT apparatus (1) according to any one of claims 1 to 10,
wherein the control unit (70) switches the display state of the display unit (75) with using a mode switching signal for switching an imaging mode between the first imaging mode and the second imaging mode as a trigger.

12. The OCT apparatus (1) according to any one of claims 1 to 11,
wherein switching of an imaging mode between the first imaging mode and the second imaging mode is automatically performed.

## Patentansprüche

1. OCT-Vorrichtung (1) umfassend:
ein optisches OCT-System (100), das einen Lichtteiler (104), der Licht von einer OCT-Lichtquelle (102) in Licht, das sich zu einem Messlichtpfad bewegt, und Licht, das sich zu einem Bezugslichtpfad bewegt, aufteilt, und einen Detektor (120) umfasst, der ein Spektruminterferenzsignal von Messlicht, das mittels des Messlichtpfads auf einen Fundus eines Subjektauges (E) geführt wird, und Bezugslicht von dem Bezugslichtpfad erfasst; und
eine Steuereinheit (70), die zum Ausgeben von OCT-Daten, die basierend auf dem Spektruminterferenzsignal erhalten werden, an eine Anzeigeeinheit (75), und zum Umschalten eines Anzeigezustands der Anzeigeeinheit (75) zwischen einem ersten Abbildungsmodus zum Erhalten von OCT-Daten über einen Fundus-Mittelbereich des Subjektauges (E) und einem zweiten Abbildungsmodus zum Erhalten von OCT-Daten über einen Weitwinkelbereich, der den Fundus-Mittelbereich und einen Fundus-Randbereich des Subjektauges (E) umfasst, konfiguriert ist, wobei
in einem Fall, in dem der erste Abbildungsmodus eingestellt ist, gibt die Steuereinheit (70) OCT-Daten eines eines vorderen (G2) und hinteren (G1) Bildbereichs in Bezug auf eine Nullverzögerungsposition (Z) auf einem Bild (G), das durch OCT-Daten gezeigt wird, an die Anzeigeeinheit (75) aus,
wobei der vordere (G2) und der hintere (G1) Bildbereich eine symmetrische Beziehung in Bezug auf die Nullverzögerungsposition (Z) umfassen,
**dadurch gekennzeichnet, dass**
in einem Fall, in dem der zweite Abbildungsmodus eingestellt ist, gibt die Steuereinheit (70) OCT-Daten sowohl des vorderen (G2) als auch des hinteren (G1) Bildbereichs in Bezug auf eine Nullverzögerungsposition (Z) auf einem durch OCT-Daten dargestellten Bild (G) an die Anzeigeeinheit (75) aus.

2. OCT-Vorrichtung (1) nach Anspruch 1,
wobei in einem Fall, in dem die Steuereinheit (70) OCT-Daten sowohl des vorderen (G2) als auch des hinteren (G1) Bildbereichs in Bezug auf eine Nullverzögerungsposition (Z) auf einem Bild (G), das durch OCT-Daten gezeigt wird, an die Anzeigeeinheit (75) ausgibt, prozessiert die Steuereinheit (70), entweder ein reales Bild (R) oder ein virtuelles Bild (M) zu entfernen, und danach prozessiert, um das andere Bild auf der Anzeigeeinheit (75) anzuzeigen.

3. OCT-Vorrichtung (1) nach Anspruch 1 oder 2, ferner umfassend
eine Speichereinheit (72), die zum Speichern von Dispersionskorrekturdaten zum Korrigieren von Dispersion in OCT-Daten konfiguriert ist,
wobei die Steuereinheit (70) das Spektrumsinterferenzsignal unter Verwendung von von der Speichereinheit (72) erhaltenen Dispersionskorrekturdaten korrigiert und Fourier-Analyse an dem korrigierten Spektrumsinterferenzsignal durchführt, um OCT-Daten zu erhalten.

4. OCT-Vorrichtung (1) nach einem der Ansprüche 1 bis 3, ferner umfassend:
eine Analyseverarbeitungseinheit (70), die zum Analysieren der OCT-Daten konfiguriert ist, um ein Analyseergebnis zu erhalten,
wobei in einem Fall, in dem die Analyseverarbeitungseinheit (70) OCT-Daten analysiert, die in dem ersten Abbildungsmodus erhalten werden, analysiert die Analyseverarbeitungseinheit (70) einen von vorderem (G2) und hinterem (G1) Bildbereich in Bezug auf eine Nullverzögerungsposition (Z) auf einem Bild (G), das durch OCT-Daten gezeigt wird, um das Analyseergebnis zu erhalten, und
in einem Fall, in dem die Analyseverarbeitungseinheit (70) OCT-Daten analysiert, die im zweiten Abbildungsmodus erhalten werden, analysiert die Analyseverarbeitungseinheit (70) sowohl den vorderen (G2) als auch den hinteren (G1) Bildbereich in Bezug auf eine Nullverzögerungsposition (Z) auf einem Bild (G), das durch OCT-Daten dargestellt wird, um das Analyseergebnis zu erhalten.

5. OCT-Vorrichtung (1) nach einem der Ansprüche 1 bis 4,
wobei die Steuereinheit (70) prozessiert, ein durch OCT-Daten dargestelltes Bild (G), das sowohl reales Bild (R) als auch virtuelles Bild (M) umfasst, auf der Anzeigeeinheit (75) anzuzeigen.

6. OCT-Vorrichtung (1) nach einem der Ansprüche 1 bis 5,
wobei die Steuereinheit (70) OCT-Daten über den Fundus-Mittelbereich, die im zweiten Abbildungsmodus erhalten werden, und OCT-Daten über den Weitwinkelbereich, der den Fundus-Mittelbereich und den Fundus-Randbereich umfasst, die im zweiten Abbildungsmodus erhalten werden, synthetisiert und prozessiert, ein Bild (G) anzuzeigen, das durch zusammengesetzte OCT-Daten auf der Anzeigeeinheit (75) dargestellt wird.

7. OCT-Vorrichtung (1) nach einem der Ansprüche 1 bis 6,
wobei die Steuereinheit (70) den Anzeigezustand der Anzeigeeinheit (75) entsprechend Umschalten eines Abbildungsmodus zwischen dem ersten Abbildungsmodus und dem zweiten Abbildungsmodus umschaltet.

8. OCT-Vorrichtung (1) nach einem der Ansprüche 1 bis 7,
wobei die Steuereinheit (70) den Anzeigezustand der Anzeigeeinheit (75) entsprechend Umschalten eines Abbildungsmodus durch Einsetzen und Zurückziehen eines Weitwinkelaufsatzes (160) zu und von dem optischen OCT-System (100) umschaltet.

9. OCT-Vorrichtung (1) nach einem der Ansprüche 1 bis 8,
wobei die Steuereinheit (70) einen Ausgabebereich von OCT-Daten auf einem Anzeigebildschirm der Anzeigeeinheit (75) entsprechend Umschalten eines Abbildungsmodus zwischen dem ersten Abbildungsmodus und dem zweiten Abbildungsmodus ändert.

10. OCT-Vorrichtung (1) nach einem der Ansprüche 1 bis 9,
wobei in einem Fall, in dem der erste Abbildungsmodus eingestellt ist, stellt die Steuereinheit (70) einen Ausgabebereich von OCT-Daten in einer Tiefenrichtung als einen ersten Ausgabebereich ein, und
in einem Fall, in dem der zweite Abbildungsmodus eingestellt ist, stellt die Steuereinheit (70) den Ausgabebereich von OCT-Daten in der Tiefenrichtung als einen zweiten Ausgabebereich ein, der breiter ist als der erste Ausgabebereich.

11. OCT-Vorrichtung (1) nach einem der Ansprüche 1 bis 10,
wobei die Steuereinheit (70) den Anzeigezustand der Anzeigeeinheit (75) unter Verwendung eines Modusumschaltsignals zum Umschalten eines Abbildungsmodus zwischen dem ersten Abbildungsmodus und dem zweiten Abbildungsmodus als Trigger umschaltet.

12. OCT-Vorrichtung (1) nach einem der Ansprüche 1 bis 11,
wobei Umschalten eines Abbildungsmodus zwischen dem ersten Abbildungsmodus und dem zweiten Abbildungsmodus automatisch durchgeführt wird.

## Revendications

1. Appareil OCT (1) comprenant :
un système optique OCT (100) qui a un diviseur de lumière (104) divisant la lumière provenant d'une source de lumière OCT (102) en une lumière se déplaçant vers un trajet de lumière de mesure et une lumière se déplaçant vers un trajet de lumière de référence, et un détecteur (120) détectant un signal d'interférence spectrale d'une lumière de mesure guidée sur le fond de l'œil (E) d'un sujet via le trajet de lumière de mesure et d'une lumière de référence provenant du trajet de lumière de référence ; et
une unité de commande (70) configurée pour délivrer en sortie des données OCT acquises sur la base du signal d'interférence spectrale à une unité d'affichage (75), et commuter un état d'affichage de l'unité d'affichage (75) entre un premier mode d'imagerie pour obtenir des données OCT sur une zone centrale de fond d'oeil de l'œil (E) de sujet et un second mode d'imagerie pour obtenir des données OCT sur une région à grand angle comprenant la zone centrale de fond d'oeil et une zone périphérique de fond d'oeil de l'œil (E) de sujet, dans lequel
dans le cas où le premier mode d'imagerie est défini, l'unité de commande (70) délivre en sortie des données OCT de l'une quelconque des zones d'image avant (G2) et arrière (G1) par rapport à une position sans retard (Z) sur une image (G) représentée par les données OCT à l'unité d'affichage (75),
dans lequel les zones d'image avant (G2) et arrière (G1) ont une relation symétrique par rapport à la position sans retard (Z),
**caractérisé en ce que**
dans le cas où le second mode d'imagerie est défini, l'unité de commande (70) délivre en sortie des données OCT des zones d'image avant (G2) et arrière (G1) par rapport à une position sans retard (Z) sur une image (G) représentée par les données OCT à l'unité d'affichage (75).

2. Appareil OCT (1) selon la revendication 1,
dans lequel, dans le cas où l'unité de commande (70) délivre en sortie des données OCT des zones d'image avant (G2) et arrière (G1) par rapport à une position sans retard (Z) sur une image (G) représentée par les données OCT à l'unité d'affichage (75), l'unité de commande (70) effectue un traitement pour supprimer l'une d'une image réelle (R) et d'une image virtuelle (M), et effectue un traitement par la suite pour afficher l'autre image sur l'unité d'affichage (75).

3. Appareil OCT (1) selon la revendication 1 ou 2, comprenant en outre :
une unité de stockage (72) configurée pour stocker des données de correction de dispersion pour corriger la dispersion dans les données OCT,
dans lequel l'unité de commande (70) corrige le signal d'interférence spectrale en utilisant des données de correction de dispersion acquises à partir de l'unité de stockage (72), et effectue une analyse de Fourier sur le signal d'interférence spectrale corrigé pour acquérir des données OCT.

4. Appareil OCT (1) selon l'une quelconque des revendications 1 à 3, comprenant en outre :
une unité de traitement d'analyse (70) configurée pour analyser les données OCT afin d'obtenir un résultat d'analyse,
dans lequel, dans le cas où l'unité de traitement d'analyse (70) analyse les données OCT acquises dans le premier mode d'imagerie, l'unité de traitement d'analyse (70) analyse l'une quelconque de zones d'image avant (G2) et arrière (G1) par rapport à une position sans retard (Z) sur une image (G) représentée par les données OCT pour acquérir le résultat d'analyse, et
dans le cas où l'unité de traitement d'analyse (70) analyse les données OCT acquises dans le second mode d'imagerie, l'unité de traitement d'analyse (70) analyse à la fois les zones d'image avant (G2) et arrière (G1) par rapport à une position sans retard (Z) sur une image (G) représentée par les données OCT pour acquérir le résultat de l'analyse.

5. Appareil OCT (1) selon l'une quelconque des revendications 1 à 4,
dans lequel l'unité de commande (70) effectue un traitement pour afficher une image (G) représentée par des données OCT comprenant à la fois une image réelle (R) et une image virtuelle (M) sur l'unité d'affichage (75).

6. Appareil OCT (1) selon l'une quelconque des revendications 1 à 5,
dans lequel l'unité de commande (70) synthétise des données OCT sur la zone centrale de fond d'oeil acquises dans le premier mode d'imagerie et des données OCT sur la région à grand angle comprenant la zone centrale de fond d'oeil et la zone périphérique de fond d'oeil acquises dans le second mode d'imagerie, et effectue un traitement pour afficher une image (G) représentée par des données OCT composites sur l'unité d'affichage (75).

7. Appareil OCT (1) selon l'une quelconque des revendications 1 à 6,
dans lequel l'unité de commande (70) commute l'état d'affichage de l'unité d'affichage (75) en fonction de la commutation d'un mode d'imagerie entre le premier mode d'imagerie et le second mode d'imagerie.

8. Appareil OCT (1) selon l'une quelconque des revendications 1 à 7,
dans lequel l'unité de commande (70) commute l'état d'affichage de l'unité d'affichage (75) en fonction de la commutation d'un mode d'imagerie par insertion et rétraction d'un accessoire à grand angle (160) dans et depuis le système optique OCT (100).

9. Appareil OCT (1) selon l'une quelconque des revendications 1 à 8,
dans lequel l'unité de commande (70) modifie une plage de sortie de données OCT sur un écran d'affichage de l'unité d'affichage (75) en fonction de la commutation d'un mode d'imagerie entre le premier mode d'imagerie et le second mode d'imagerie.

10. Appareil OCT (1) selon l'une quelconque des revendications 1 à 9,
dans lequel, dans le cas où le premier mode d'imagerie est défini, l'unité de commande (70) définit une plage de sortie de données OCT dans une direction de profondeur comme première plage de sortie, et
dans le cas où le second mode d'imagerie est défini, l'unité de commande (70) définit la plage de sortie de données OCT dans la direction de profondeur comme seconde plage de sortie qui est plus large que la première plage de sortie.

11. Appareil OCT (1) selon l'une quelconque des revendications 1 à 10, dans lequel l'unité de commande (70) commute l'état d'affichage de l'unité d'affichage (75) en utilisant un signal de commutation de mode pour commuter un mode d'imagerie entre le premier mode d'imagerie et le second mode d'imagerie comme déclencheur.

12. Appareil OCT (1) selon l'une quelconque des revendications 1 à 11,
dans lequel la commutation d'un mode d'imagerie entre le premier mode d'imagerie et le second mode d'imagerie est effectuée automatiquement.
